(19) Europäisches Patentamt
European Patent Office
Office européen des brevets

(11) Veröffentlichungsnummer : **0 107 198**
**B1**

(12) # EUROPÄISCHE PATENTSCHRIFT

(45) Veröffentlichungstag der Patentschrift : **08.07.87**

(51) Int. Cl.⁴ : **C 08 F 2/50**, G 03 C 1/68, C 07 C 49/82

(21) Anmeldenummer : **83110568.9**

(22) Anmeldetag : **18.12.78**

(60) Veröffentlichungsnummer der früheren Anmeldung nach Art. 76 EPÜ : **0003002**

(54) **Verwendung von 2-Hydroxy-2-methyl(p-chlorpropiophenon) als Photoinitiator.**

(30) Priorität : **22.12.77 CH 15884/77**

(43) Veröffentlichungstag der Anmeldung : **02.05.84 Patentblatt 84/18**

(45) Bekanntmachung des Hinweises auf die Patenterteilung : **08.07.87 Patentblatt 87/28**

(84) Benannte Vertragsstaaten : **BE CH DE FR GB IT NL SE**

(56) Entgegenhaltungen :
**DE-A- 2 722 264**
**DE-A- 2 808 459**
**CHEMICAL ABSTRACTS, Band 55, 1961, Spalte 441 unten - Spalte 442 bis d, Columbus, Ohio, US., T.I. TEMNIKOVA u.a.: "Cyclic acetals of hydroxycarbonyl compounds. X: Methyllactolides of dimethyl-p-anisoyl- and dimethyl-p-chlorobenzoylcarbinols and their transformations"**

(73) Patentinhaber : **CIBA-GEIGY AG**
**Klybeckstrasse 141**
**CH-4002 Basel (CH)**

(72) Erfinder : **Felder, Louis, Dr.**
**Riehenring 5**
**CH-4058 Basel (CH)**
Erfinder : **Kirchmayr, Rudolf, Dr.**
**Ettingerstrasse 9**
**CH-4147 Aesch (CH)**
Erfinder : **Hüsler, Rinaldo, Dr.**
**Belchenstrasse 12**
**CH-4054 Basel (CH)**

**EP 0 107 198 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die Verwendung der im Titel genannten Verbindung als Initiator für die Photopolymerisation ungesättigter Verbindungen.

Photochemische Polymerisationsprozesse haben in der Technik erhebliche Bedeutung erlangt, vor allem in solchen Fällen, wo dünne Schichten in kurzer Zeit gehärtet werden müssen, wie z. B. bei der Härtung von Lacküberzügen oder bei der Trocknung von Druckfarben. Die UV-Bestrahlung in Gegenwart von Photoinitiatoren zeigt gegenüber herkömmlichen Härtungsverfahren eine Reihe von Vorteilen, deren wichtigster wohl die hohe Geschwindigkeit der Photohärtung ist. Die Geschwindigkeit ist stark vom verwendeten Photoinitiator abhängig und es hat nicht an Versuchen gefehlt, die herkömmlichen Initiatoren durch immer bessere und wirksamere Verbindungen zu ersetzen. Zu den wirksamsten Photoinitiatoren gehören Derivate des Benzoins, vor allem Benzoin-äther wie sie beispielsweise in den Brit. Patentschriften 1 254 231 und 1 156 460 oder im Schweiz. Patent 511 902 beschrieben werden, sowie Dialkoxyacetophenone und Benzil-monoketale, wie sie beispielsweise in der DT-OS 2 261 383 oder 2 365 497 beschrieben sind.

Nachteile, die diesen bekannten Photoinitiatoren anhaften, sind z. T. ungenügende Dunkellagerstabilität der mit solchen Initiatoren vermischten photopolymerisierbaren Systeme. Einige Benzoinderivate neigen zur Vergilbung der gehärteten Massen. Andere Initiatoren besitzen unzureichende Reaktivität, was sich in relativ langen Härtungszeiten äussert, oder sie sind in den photopolymerisierbaren Systemen zu wenig löslich oder sie werden durch Luftsauerstoff rasch inaktiviert. In der Technik besteht daher ein Bedarf an Photoinitiatoren, die im Substrat gut löslich sind und bei guter Dunkellagerstabilität die Photopolymerisation rascher auslösen und eine höhere Polymerausbeute pro Zeiteinheit ergeben als die bekannten Photoinitiatoren. Durch die Verwendung solcher verbesserter Photoinitiatoren würden sich die kostspieligen industriellen UV-Bestrahlungsanlagen besser ausnützen lassen.

Es wurde gefunden, dass 2-Hydroxy-2-methyl-p-chlorpropiophenon die geforderten Eigenschaften besitzt, vor allem eine schnelle Polymerisation bewirkt und die geschilderten Nachteile nicht oder in wesentlich geringerem Masse aufweist als die bekannten Photoinitiatoren.

Diese Verbindung entspricht der Formel

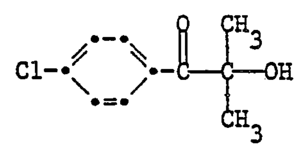

und kann aus p-Chlorisobutyrophenon durch Bromierung und anschliessende Behandlung mit $NaOCH_3$ und anschliessende Hydrolyse hergestellt werden gemäss folgendem Schema :

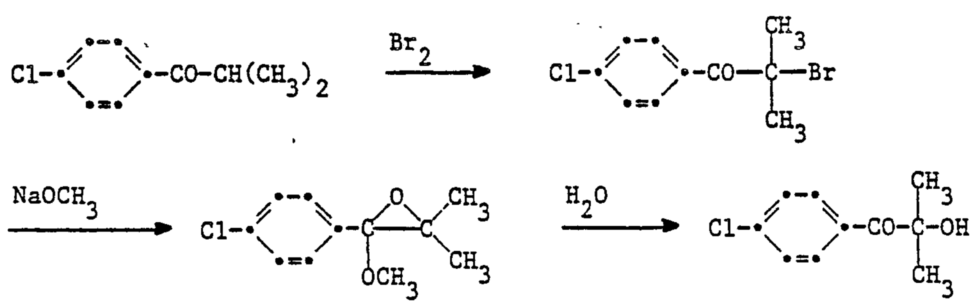


Erfindungsgemäss kann die Verbindung als Initiator zur Photopolymerisation von ungesättigten Verbindungen verwendet werden.

Solche Verbindungen sind beispielsweise ungesättigte Monomere wie Ester von Acryl- oder Methacrylsäure, z. B. Methyl-, Aethyl-, n- oder tert. Butyl-, Isooctyl- oder Hydroxyäthylacrylat, Methyl- oder Aethylmethacrylat, Aethylendiacrylat, Neopentyl-diacrylat, Trimethylolpropantrisacrylat, Pentaerythrittetraacrylat oder Pentaerythrittrisacrylat ; Acrylnitril, Methacrylnitril, Acrylamid, Methacrylamid, N-substituierte (Meth)acrylamide ; Vinylester wie z. B. Vinylacetat, -propionat, -acrylat oder -succinat ; sonstige Vinylverbindungen wie Vinyläther, Styrol, Alkylstyrole, Halogenstyrole, Divinylbenzol, Vinylnaphthalin, N-Vinylpyrrolidon, Vinylchlorid oder Vinylidenchlorid ; Allylverbindungen wie Diallylphthalat, Diallylmaleat, Triallylisocyanurat, Triallylphosphat oder Aethylenglycol-diallyläther und die Mischungen von solchen ungesättigten Monomeren.

Photopolymerisierbare Verbindungen sind weiterhin ungesättigte Oligomere oder Polymere und deren Mischungen mit ungesättigten Monomeren. Hierzu zählen thermoplastische Harze, die ungesättigte Gruppen wie Fumarsäureester, Allylgruppen oder Acrylat- oder Methacrylatgruppen enthalten. Meist sind diese ungesättigten Gruppen über funktionelle Gruppen an die Hauptkette dieser linearen Polymeren

gebunden. Grosse Bedeutung haben Gemische von Oligomeren mit einfach und mehrfach ungesättigten Monomeren. Beispiele für solche Oligomere sind ungesättigte Polyester, ungesättigte Acrylharze und Isocyanat- oder Epoxid- modifizierte Acrylatoligomere sowie Polyätheracrylatoligomere. Beispiele für mehrfach ungesättigte Verbindungen sind vor allem die Acrylate von Diolen und Polyolen, z. B. Hexamethylen-diacrylat oder Pentaerythrittetracrylat. Auch als einfach ungesättigte Monomere werden Acrylate bevorzugt wie z. B. Butylacrylat, Phenylacrylat, Benzylacrylat, 2-Aethyl-hexylacrylat oder 2-Hydroxypropylacrylat. Man hat es durch Auswahl aus den verschiedenen Vertretern der drei Komponenten in der Hand, die Konsistenz des unpolymerisierten Gemisches sowie die Plastizität des polymerisierten Harzes zu variieren.

Neben diesen Dreikomponenten-Gemischen spielen bei den Polyesterharzen vor allem Zweikomponenten-Gemische eine grosse Rolle. Diese bestehen meist aus einem ungesättigten Polyester und einer Vinylverbindung. Die ungesättigten Polyester sind oligomere Veresterungsprodukte mindestens einer ungesättigten Dicarbonsäure wie z. B. Malein-, Fumar- oder Citraconsäure, und meist mindestens einer gesättigten Dicarbonsäure, wie z. B. Phthalsäure, Bernsteinsäure, Sebazinsäure oder Isophthalsäure, mit Glykolen wie z. B. Aethylenglykol, Propandiol-1,2, Di- oder Triäthylenglykol oder Tetramethylenglykol, wobei zur Modifizierung meist auch Monocarbonsäuren und Monoalkohole mitverwendet werden. Diese ungesättigten Polyester werden üblicherweise in einer Vinyl- oder Allylverbindung gelöst, bevorzugt wird hierfür Styrol verwendet.

Photopolymerisierbare Systeme, wie sie für die verschiedenen Zwecke verwendet werden, enthalten meist ausser den photopolymerisierbaren Verbindungen und dem Photoinitiator eine Reihe sonstiger Zusätze. So ist es vielfach üblich, thermische Inhibitoren zuzusetzen, die vor allem während der Herstellung der Systeme durch Mischen der Komponenten vor einer vorzeitigen Polymerisation schützen sollen.

Zur Erhöhung der Dunkellagerstabilität können Kupferverbindungen wie Kupfernaphthenat, -stearat, oder -octoat, Phosphorverbindungen wie Triphenylphosphin, Tributylphosphin, Triäthylphosphit, Triphenylphosphit oder Tribenzylphosphat, quartäre Ammoniumverbindungen wie Tetramethylammoniumchlorid oder Trimethyl-benzylammoniumchlorid oder Hydroxylaminderivate wie z. B N-Diäthylhydroxylamin, zugesetzt werden.

Photopolymerisierbare Systeme enthalten weiterhin — je nach Verwendungszweck — Füllstoffe wie Kieselsäure, Talkum oder Gips, Pigmente, Farbstoffe, Fasern, Thixotropiemittel oder Verlaufhilfsmittel.

Ferner können auch Kombinationen mit bekannten Photoinitiatoren, wie Benzoinäthern, Dialkoxyacetophenonen oder Benzilketalen, verwendet werden.

Besonders für die Photopolymerisation von dünnen Schichten und Druckfarben können Kombinationen des erfindungsgemässen Photoinitiators mit Aminen und/oder aromatischen Ketonen verwendet werden. Beispiele für Amine sind Triäthylamin, N-Methyldiäthanolamin, N-Dimethyläthanolamin oder p-Dimethylaminobenzoesäureester. Beispiele für Ketone sind Benzophenon, substituierte Benzophenonderivate, Michler's Ketone, Anthrachinon und Anthrachinonderivate, sowie Thioxanthon und dessen Derivate.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet ist der erfindungsgemässe Initiator auch für photohärtbare Systeme zur Herstellung von Druckplatten. Hierbei werden z. B. Gemische von löslichen linearen Polyamiden mit photopolymerisierbaren Monomeren, beispielsweise Acrylamide, und einem Photoinitiator verwendet. Filme oder Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der UV-Härtung ist die Metallbeschichtung, beispielsweise bei der Lackierung von Blechen für Tuben, Dosen oder Flaschenverschlüssen, sowie die UV-Härtung von Kunststoffbeschichtungen, beispielsweise von Fussboden- oder Wandbelägen auf PVC-Basis.

Beispiele für die UV-Härtung von Papierbeschichtungen sind die farblose Lackierung von Etiketten, Schallplatten-Hüllen oder Buchumschlägen.

Der Photoinitiator wird für die angeführten Anwendungsgebiete zweckmässig in Mengen von 0,1 bis 20 Gew.-%, vorzugsweise etwa 0,5 bis 5 Gew.-%, bezogen auf das photopolymerisierbare bzw. vernetzbare System, angewendet. Unter System ist hierbei das Gemisch aus der photopolymerisierbaren bzw. vernetzbaren Verbindung, dem Photoinitiator und den sonstigen Füll- und Zusatzstoffen gemeint wie es in der jeweiligen Anwendung verwendet wird.

Der Zusatz des Photoinitiators zu den photopolymerisierbaren Systemen geschieht im allgemeinen durch einfaches Einrühren, da die meisten dieser Systeme flüssig oder gut löslich sind. Meist kommt es zu einer Lösung des Initiators, wodurch dessen gleichmässige Verteilung sowie die Transparenz der Polymerisate gewährleistet ist.

Die Polymerisation erfolgt nach den bekannten Methoden der Photopolymerisation durch Bestrahlung mit Licht, das reich an kurzwelliger Strahlung ist. Als Lichtquellen sind z. B. Quecksilbermitteldruck-, -hochdruck- und -niederdruckstrahler, sowie superaktinische Leuchtstoffröhren geeignet, deren Emissionsmaxima im Bereich zwischen 250 und 400 nm liegen.

Beispiel

Härtung eines Acrylatharzes

Eine Harzmischung aus 70 Teilen Ebecryl 593 (Polyesteracrylat der Firma UCB, Belgien), 30 Teilen Trimethylolpropantrisacrylat, 0,5 Teilen ByK 300 (Verlaufshilfsmittel der Firma ByK-Mallinckrodt, BRD), und 3 Teilen des erfindungsgemässen Photoinitiators wird mit einem Aufziehrahmen in einer Schichtdicke von 30-40 μ auf Glasplatten aufgegetragen. Nach einer kurzen Abluftzeit wird mit einem UV-Laborgerät (Modell PPG/Q.C-Prozessor) mit einer UV-Lampe von 80 Watt/cm ausgehärtet. Nach der UV-Härtung wird 1/2 Stunde im Normklima gelagert und anschliessend die Pendelhärte nach König bestimmt. Die Pendelhärte beträgt bei einer Transportgeschwindigkeit von 10 m/min 161 s und bei einer Transportgeschwindigkeit von 25 m/min 152 s.

**Patentanspruch**

Verwendung von 2-Hydroxy-2-methyl-(p-chlorpropiophenon) der Formel

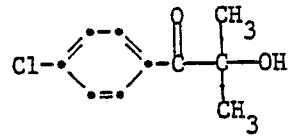

als Initiator für die Photopolymerisation ungesättigter Verbindungen.

**Claim**

Use of 2-hydroxy-2-methyl-(p-chloropropiophenone) of the formula

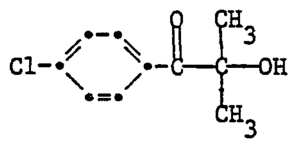

as initiator for the photopolymerisation of unsaturated compounds.

**Revendication**

Application de l'hydroxy-2 méthyl-2 (p-chloro-propiophénone), corps qui répond à la formule suivante :

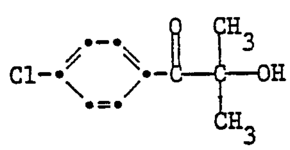

comme amorceur pour la photopolymérisation de composés insaturés.